# EUROPEAN PATENT SPECIFICATION

(11) **EP 0 797 437 B1**
(45) Date of publication and mention of the grant of the patent: **18.04.2001**
(21) Application number: 95940063.1
(22) Date of filing: 20.11.1995
(51) Int. Cl.: A61K 31/40, A61K 31/44

(54) **IMMUNOTHERAPEUTIC IMIDES/AMIDES AND THEIR USE FOR REDUCING LEVELS OF TNFalpha**
IMMUNOTHERAPEUTISCHE IMIDE/AMIDE UND IHRE VERWENDUNG ZUR VERMINDERUNG DES TNFalpha-SPIEGELS
IMIDES/AMIDES IMMUNOTHERAPEUTIQUES ET LEUR UTILISATION POUR REDUIRE LE NIVEAU DU FACTEUR alpha DE NECROSE DE TUMEURS

(30) Priority: 30.12.1994 US 366667
(43) Date of publication of application: 01.10.1997
(73) Proprietor: CELGENE CORPORATION, Warren New Jersey 07059 (US)
(72) Inventor: MULLER, George, Bridgewater, NJ 08807 (US); SHIRE, Mary, North Plainfield, NJ 07060 (US); STIRLING, David I., Branchburg, NJ 08876 (US)
(74) Representative: Howard, Paul Nicholas
(86) International application number: US9515119
(87) International publication number: WO9620705

(56) References cited:
- WO-A-92/14455
- WO-A-95/01348
- DATABASE MEDLINE US NATIONAL LIBRARY OF MEDICINE (NLM), BETHESDA, MD, US WANG L ET AL '[Studies on antiviral agents: synthesis of tai-ding-an analogs]' & YAO HSUEH HSUEH PAO, 1994, 29 (6) P427-32, CHINA,
- BIOLOGICAL AND PHARMACEUTICAL RESEARCH BULLETIN, vol. 17, no. 9, September 1994 pages 1313-1315, SASAKI, K. ET AL 'Enhancement of 12-O-tetradecanoylphorbol-13-acetate- induced tumor necrosis factor alpha production by phenethylphthalimide analogs'
- BIOCHEMICAL AND BIOPHYSICAL RESEARCH COMMUNICATIONS, vol. 193, no. 1, 1993 pages 277-283, SUZUKI, Y.J. ET AL 'Inhibition of NF-kB activation by vitamin E derivatives' cited in the application
- JOURNAL OF ACQUIRED IMMUNE DEFICIENCY SYNDROMES, vol. 6, no. 7, 1993 pages 778-786, BISWAS, D.K. ET AL 'Pentoxifylline inhibits HIV-1 LTR-driven gene expression by blocking NF-kB action' cited in the application

## Description

### BACKGROUND OF THE INVENTION

The present invention relates a method of reducing levels of TNFα in a mammal and to compounds and compositions useful therein.

TNFα, or tumor necrosis factor α, is a cytokine which is released primarily by mononuclear phagocytes in response to various immunostimulators. When administered to animals or humans it causes inflammation, fever, cardiovascular effects, hemorrhage, coagulation and acute phase responses similar to those seen during acute infections and shock states.

Excessive or unregulated TNFα production has been implicated in a number of disease conditions. These include endotoxemia and/or toxic shock syndrome {Tracey *et al.*, *Nature* **330**, 662-664 (1987) and Hinshaw *et al.*, *Circ. Shock* **30**, 279-292 (1990)}; cachexia {Dezube *et al., Lancet,* **335**(8690), 662 (1990)}; and Adult Respiratory Distress Syndrome where TNFα concentration in excess of 12,000 pg/milliliter have been detected in pulmonary aspirates from ARDS patients {Millar *et al.*, *Lancet* **2**(8665), 712-714 (1989)}. Systemic infusion of recombinant TNFα also resulted in changes typically seen in ARDS {Ferrai-Baliviera *et al., Arch. Surg.* **124**(12), 1400-1405 (1989)}.

TNFα appears to be involved in bone resorption diseases, including arthritis where it has been determined that when activated, leukocytes will produce a bone-resorbing activity, and data suggest that TNFα contributes to this activity. {Bertolini *et al*., *Nature* **319**, 516-518 (1986) and Johnson *et al*., *Endocrinology* **124**(3), 1424-1427 (1989).} It has been determined that TNFα stimulates bone resorption and inhibits bone formation *in vitro* and *in vivo* through stimulation of osteoclast formation and activation combined with inhibition of osteoblast function. Although TNFα may be involved in many bone resorption diseases, including arthritis, the most compelling link with disease is the association between production of TNFα by tumor or host tissues and malignancy associated hypercalcemia {*Calci. Tissue Int. (US)* **46**(Suppl.), S3-10 (1990)}. In Graft versus Host Reaction, increased serum TNFα levels have been associated with major complication following acute allogenic bone marrow transplants {Holler *et al., Blood,* **75**(4), 1011-1016 (1990)}.

Cerebral malaria is a lethal hyperacute neurological syndrome associated with high blood levels of TNFα and the most severe complication occurring in malaria patients. Levels of serum TNFα correlated directly with the severity of disease and the prognosis in patients with acute malaria attacks {Grau *et al., N*. *Engl. J*. *Med*. **320**(24), 1586-1591 (1989)}.

TNFα also plays a role in the area of chronic pulmonary inflammatory diseases. The deposition of silica particles leads to silicosis, a disease of progressive respiratory failure caused by a fibrotic reaction. Antibody to TNFα completely blocked the silica-induced lung fibrosis in mice {Pignet *et al*., *Nature,* **344**:245-247 (1990)}. High levels of TNFα production (in the serum and in isolated macrophages) have been demonstrated in animal models of silica and asbestos induced fibrosis {Bissonnette *et al*., *Inflammation* **13**(3), 329-339 (1989)}. Alveolar macrophages from pulmonary sarcoidosis patients have also been found to spontaneously release massive quantities of TNFα as compared with macrophages from normal donors {Baughman *et al.*, *J. Lab. Clin. Med.* **115**(1), 36-42 (1990)}.

TNFα is also implicated in the inflammatory response which follows reperfusion, called reperfusion injury, and is a major cause of tissue damage after loss of blood flow {Vedder *et al., PNAS* **87**, 2643-2646 (1990)}. TNFα also alters the properties of endothelial cells and has various pro-coagulant activities, such as producing an increase in tissue factor pro-coagulant activity and suppression of the anticoagulant protein C pathway as well as down-regulating the expression of thrombomodulin {Sherry *et al*., *J*. *Cell Biol*. **107**, 1269-1277 (1988)}. TNFα has pro-inflammatory activities which together with its early production (during the initial stage of an inflammatory event) make it a likely mediator of tissue injury in several important disorders including but not limited to, myocardial infarction, stroke and circulatory shock. Of specific importance may be TNFα-induced expression of adhesion molecules, such as intercellular adhesion molecule (ICAM) or endothelial leukocyte adhesion molecule (ELAM) on endothelial cells {Munro *et al*., *Am*. *J*. *Path.* **135**(1), 121-132 (1989)}.

Moreover, it now is known that TNFα is a potent activator of retrovirus replication including activation of HIV-1. {Duh *et al., Proc. Nat. Acad*. *Sci.* **86**, 5974-5978 (1989); Poll *et al., Proc. Nat. Acad. Sci*. **87**, 782-785 (1990); Monto *et al., Blood* **79**, 2670 (1990); Clouse *et al., J*. *Immunol.* **142**, 431-438 (1989); Poll *et al., AIDS Res. Hum*. *Retrovirus,* 191-197 (1992)}. AIDS results from the infection of T lymphocytes with Human Immunodeficiency Virus (HIV). At least three types or strains of HIV have been identified, *i*.*e*., HIV-1, HIV-2 and HIV-3. As a consequence of HIV infection, T-cell mediated immunity is impaired and infected individuals manifest severe opportunistic infections and/or unusual neoplasms. HIV entry into the T lymphocyte requires T lymphocyte activation. Other viruses, such as HIV-1, HIV-2 infect T lymphocytes after T cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication. Cytokines, specifically TNFα, are implicated in activated T-cell mediated HIV protein expression and/or virus replication by playing a role in maintaining T lymphocyte activation. Therefore, interference with cytokine activity such as by prevention or inhibition of cytokine production, notably TNFα, in an HIV-infected individual aids in limiting the maintenance of T lymphocyte caused by HIV infection.

Monocytes, macrophages, and related cells, such as kupffer and glial cells, have also been implicated in maintenance of the HIV infection. These cells, like T cells, are targets for viral replication and the level of viral replication is dependent upon the activation state of the cells. {Rosenberg *et al*., *The Immunopathogenesis of HIV Infection,* Advances in Immunology, **57** (1989)}. Cytokines, such as TNFα, have been shown to activate HIV replication in monocytes and/or macrophages {Poli *et al. Proc. Natl. Acad*. *Sci*., **87**, 782-784 (1990)}, therefore, prevention or inhibition of cytokine production or activity aids in limiting HIV progression as stated above for T cells. Additional studies have identified TNFα as a common factor in the activation of HIV *in vitro* and has provided a clear mechanism of action via a nuclear regulatory protein found in the cytoplasm of cells (Osborn, *et al*., *PNAS* **86**, 2336-2340). This evidence suggests that a reduction of TNFα synthesis may have an antiviral effect in HIV infections, by reducing the transcription and thus virus production.

AIDS viral replication of latent HIV in T cell and macrophage lines can be induced by TNFα {Folks *et al*., *PNAS* **86**, 2365-2368 (1989)}. A molecular mechanism for the virus inducing activity is suggested by TNFα's ability to activate a gene regulatory protein (NFκB) found in the cytoplasm of cells, which promotes HIV replication through binding to a viral regulatory gene sequence (LTR) {Osborn *et al.*, *PNAS* **86**, 2336-2340 (1989)}. TNFα in AIDS and cancer associated cachexia is suggested by elevated serum TNFα and high levels of spontaneous TNFα production in peripheral blood monocytes from patients {Wright *et al*. *J. Immunol.* **141**(1), 99-104 (1988)}. {Eur J. Gastroen Hepat, 6(9), 821-829 (1994)}. {J. Exp. Med., 1121-1227 (1988)}.

TNFα has been implicated in various roles with other viral infections, such as the cytomegalia virus (CMV), influenza virus, adenovirus, and the herpes family of viruses for similar reasons as those noted.

Preventing or inhibiting the production or action of TNFα is, therefore, predicted to be a potent therapeutic strategy for many inflammatory, infectious, immunological or malignant diseases. These include but are not restricted to septic shock, sepsis, endotoxic shock, hemodynamic shock and sepsis syndrome, post ischemic reperfusion injury, malaria, mycobacterial infection, meningitis, psoriasis, congestive heart failure, fibrotic disease, cachexia, graft rejection, cancer, autoimmune disease, opportunistic infections in AIDS, rheumatoid arthritis, rheumatoid spondylitis, osteoarthritis, other arthritic conditions, Crohn's disease, ulcerative colitis, multiple sclerosis, systemic lupus erythrematosis, ENL in leprosy, radiation damage, and hyperoxic alveolar injury. Efforts directed to the suppression of the effects of TNFα have ranged from the utilization of steroids such as dexamethasone and prednisolone to the use of both polyclonal and monoclonal antibodies {Beutler *et al., Science* **234**, 470-474 (1985); WO 92/11383}. (Clinical and Experimental Rheumatology 1993, 11 (Suppl. 8), 5173-5175). (PNAS 1992, 89, 9784-88). (Annals of the Rheumatic Diseases 1990, 49, 480-486).

The nuclear factor κB (NFκB) is a pleiotropic transcriptional activator (Lenardo, *et al*. Cell 1989, 58, 227-29). NFκB has been implicated as a transcriptional activator in a variety of disease and inflammatory states and is thought to regulate cytokine levels including but not limited to TNFα and also to be an activator of HIV transcription (Dbaibo, *et al*. J. Biol. Chem. 1993, 17762-66; Duh *et al*. Proc. Natl. Acad. Sci. 1989, 86, 5974-78; Bachelerie *et al.* Nature 1991, 350, 709-12; Boswas *et al*. J.. Acquired Immune Deficiency Syndrome 1993, 6, 778-786; Suzuki *et al*. Biochem. And Biophys. Res. Comm. 1993, 193, 277-83; Suzuki *et al*. Biochem. And Biophys. Res Comm. 1992, 189, 1709-15; Suzuki *et al.* Biochem. Mol. Bio. Int. 1993, 31(4), 693-700; Shakhov *et al*. 1990, 171, 35-47; and Staal *et al.* Proc. Natl. Acad. Sci. USA 1990, 87, 9943-47). Thus, inhibition of NFκB binding can regulate transcription of cytokine gene(s) and through this modulation and other mechanisms be useful in the inhibition of a multitude of disease states. The compounds claimed in this patent can inhibit the action of NFκB in the nucleus and thus are useful in the treatment of a variety of diseases including but not limited to rheumatoid arthritis, rheumatoid spondylitis, osteoarthritis, other arthritic conditions, septic shock, septis, endotoxic shock, graft versus host disease, wasting, Crohn's disease, ulcerative colitis, multiple sclerosis, systemic lupus erythrematosis, ENL in leprosy, HIV, AIDS, and opportunistic infections in AIDS.

TNFα and NFκB levels are influenced by a reciprocal feedback loop. As noted above, the compounds of the present invention affect the levels of both TNFα and NFκB. It is not known at this time, however, how the compounds of the present invention regulate the levels of TNFα, NFκB, or both.

### Detailed Description

The present invention is based on the discovery that a class of non-polypeptide imides/amides more fully described herein appear to inhibit the action of TNFα.

The present invention pertains to compounds of the formula: 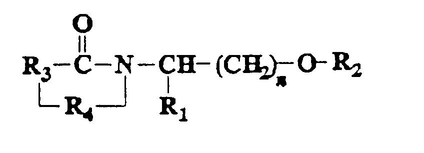
in which R¹ is (*i*) straight, branched, or cyclic, substituted or unsubstituted alkyl of 1 to 12 carbon atoms, (*ii*) phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or halo;
R² is -H, alkyl of 1 to 8 carbon atoms;
R³ is i) ethylene, ii) vinylene, iii) a branched alkylene of 3 to 10 carbon atoms, iv) a branched alkenylene of 3 to 10 carbon atoms, v) cycloalkylene of 4 to 9 carbon atoms unsubstituted or substituted with 1 or more substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, vi) cycloalkenylene of 4 to 9 carbon atoms unsubstituted or substituted with 1 or more substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, or vii) o-phenylene unsubstituted or substituted with 1 or more substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo;
R⁴ is -CX- or -CH₂-;
X is O or S;
   and,
n is 0,1,2, or 3.

A first preferred subclass of Formula II pertains to compounds in which R³ is *o*-phenylene ;
R¹ is 3,4-diethoxyphenyl
R⁴ is -CO-.

Typical compounds of this invention include 3-phthalimido-3-(3',4'-dimethoxyphenyl)propan-1-ol; 2-phthalimido-2-(3',4'-dimethoxyphenyl)ethanol; 3-phthalimido-3-(3',4'-diethoxyphenyl)propan-1-ol; 3-phthalimido-3-(3',4'-dimethoxyphenyl)-1-methoxypropane; 2-phthalimido-2-(3',4'-dimethoxyphenyl)-1-methoxyethane; 3-phthalimido-3-(3',4'-diethoxyphenyl)-1-methoxypropane; 3-phthalimido-3-(3',4'-dimethoxyphenyl)-1-ethoxypropane; 3-phthalimido-3-(3',4'-dimethoxyphenyl)-1-(3-pyridylmethoxy)propane; 3-phthalimido-3-(3',4'-diethoxyphenyl)-1-(3-pyridylmethoxyl)propane; 3-phthalimido-3-napthylpropan-1-ol; 3-phthalimido-3-(3',4'-diethylphenyl)propan-1-ol; 3-phthalimido-3-(3',4'-dipropylphenyl)propan-1-ol; 3-phthalimido-3-(3',4'-diethylphenyl)-1-methoxypropane; 3-phthalimido-3-(3',4'-diethoxyphenyl)-1-ethoxypropane; 3-phthalimido-3-cyclohexyl-1-methoxypropane; 3-phthalimido-3-(3',4'-diethylcyclohexyl)-1-methoxypropane; 3-(1'-oxoisoindolinyl)-3-(3',4'-dimethoxyphenyl)propan-1-ol; 2-(1'-oxoisoindolinyl)-2-(3',4'-dimethoxyphenyl)ethanol; 3-(1'-oxoisoindolinyl)-3-(3',4'-diethoxyphenyl)propan-1-ol; 3-(1'-oxoisoindolinyl-3-(3',4'-dimethoxyphenyl)-1-methoxypropane; 2-(1'-oxoisoindolinyl)-2-(3',4'-dimethoxyphenyl)-1-methoxyethane; 3-(1'oxoisoindolinyl)-3-(3',4'-diethoxyphenyl)-1-methoxypropane; 3-(1'-oxoisoindolinyl)-3-(3',4'-dimethoxyphenyl)-1-ethoxypropane; 3-(1'-oxoisoindolinyl)-3-(3',4'-dimethoxyphenyl)-1-(3-pyridylmethoxy)propane; 3-(1'-oxoisoindolinyl)-3-(3',4'-diethoxyphenyl)-1-(3-pyridylmethoxyl)propane; 3-(1'-oxoisoindolinyl)-3-napthylpropan-1-ol; 3-(1'-oxoisoindolinyl)-3-(3',4'-diethylphenyl)propan-1-ol; 3-(1'-oxoisoindolinyl)-3-(3',4'-dipropylphenyl)propan-1-ol; 3-(1'-oxoisoindolinyl)-3-(3',4'-diethylphenyl)-1-methoxypropane; 3-(1'-oxoisoindolinyl)-3-(3',4'-diethoxyphenyl)-1-ethoxypropane.

The term alkyl as used herein denotes a univalent saturated branched or straight hydrocarbon chain. Unless otherwise stated, such chains can contain from 1 to 18 carbon atoms. Representative of such alkyl groups are methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl, undecyl, dodecyl, tridecyl, tetradecyl, pentadecyl, hexadecyl, heptadecyl, octadecyl, and the like. When qualified by "lower", the alkyl group will contain from 1 to 6 carbon atoms. The same carbon content applies to the parent term "alkane" and to derivative terms such as "alkoxy".

The compounds can be used, under the supervision of qualified professionals, to inhibit the undesirable effects of TNFα. The compounds can be administered orally, rectally, or parenterally, alone or in combination with other therapeutic agents including antibiotics, steroids, etc., to a mammal in need of treatment. Oral dosage forms include tablets, capsules, dragees, and similar shaped, compressed pharmaceutical forms. Isotonic saline solutions containing 20-100 milligrams/milliliter can be used for parenteral administration which includes intramuscular, intrathecal, intravenous and intra-arterial routes of administration. Rectal administration can be effected through the use of suppositories formulated from conventional carriers such as cocoa butter.

Dosage regimens must be titrated to the particular indication, the age, weight, and general physical condition of the patient, and the response desired but generally doses will be from about 1 to about 500 milligrams/day as needed in single or multiple daily administration. In general, an initial treatment regimen can be copied from that known to be effective in interfering with TNFα activity for other TNFα mediated disease states by the compounds of the present invention. Treated individuals will be regularly checked for T cell numbers and T4/T8 ratios and/or measures of viremia such as levels of reverse transcriptase or viral proteins, and/or for progression of cytokine-mediated disease associated problems such as cachexia or muscle degeneration. If no effect is found following the normal treatment regimen, then the amount of cytokine activity interfering agent administered is increased, *e.g.*, by fifty percent a week.

The compounds of the present invention also can be used topically in the treatment or prophylaxis of topical disease states mediated or exacerbated by excessive TNFα production, respectively, such as viral infections, such as those caused by the herpes viruses, or viral conjunctivitis, *etc.*

The compounds also can be used in the veterinary treatment of mammals other than humans in need of prevention or inhibition of TNFα production. TNFα mediated diseases for treatment, therapeutically or prophylactically, in animals include disease states such as those noted above, but in particular viral infections. Examples include feline immunodeficiency virus, equine infectious anaemia virus, caprine arthritis virus, visna virus, and maedi virus, as well as other lentiviruses.

Certain of these compounds possess centers of chirality and can exist as optical isomers. Both the racemates of these isomers and the individual isomers themselves, as well as diastereomers when there are two chiral centers, are within the scope of the present invention. The racemates can be used as such or can be separated into their individual isomers mechanically as by chromatography using a chiral absorbant. Alternatively, the individual isomers can be prepared in chiral form or separated chemically from a mixture by forming salts with a chiral acid, such as the individual enantiomers of 10-camphorsulfonic acid, camphoric acid, alpha-bromocamphoric acid, methoxyacetic acid, tartaric acid, diacetyltartaric acid, malic acid, pyrrolidone-5-carboxylic acid, and the like, and then freeing one or both of the resolved bases, optionally repeating the process, so as to obtain either or both substantially free of the other; *i*.*e*., in a form having an optical purity of >95%.

Prevention or inhibition of production of TNFα by these compounds can be conveniently assayed using anti-TNFα antibodies. For example, plates (Nunc Immunoplates, Roskilde, DK) are treated with 5 µg/milliliter of purified rabbit anti-TNFα antibodies at 4°C for 12 to 14 hours. The plates then are blocked for 2 hours at 25°C with PBS/0.05% Tween containing 5 milligrams/milliliter BSA. After washing, 100 µL of unknowns as well as controls are applied and the plates incubated at 4°C for 12 to 14 hours. The plates are washed and assayed with a conjugate of peroxidase (horseradish) and mouse anti-TNFα monoclonal antibodies, and the color developed with o-phenylenediamine in phosphate-citrate buffer containing 0.012% hydrogen peroxide and read at 492 nm.

The compounds can be prepared using methods which are known in general for the preparation of imides. General reaction schemes include the reaction of the substituted amine with either phthalic anhydride, N-carbethoxyphthalimide, or phthalic dicarboxaldehyde as illustrated by the formulas:

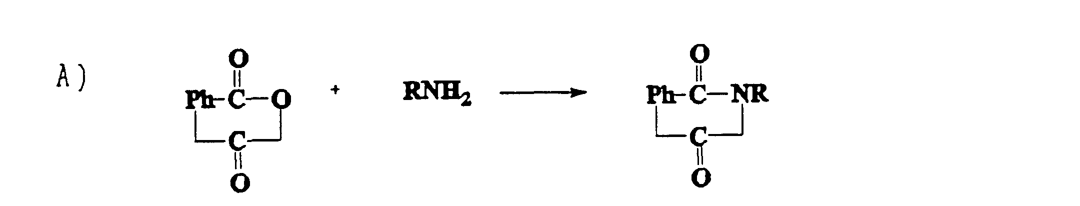

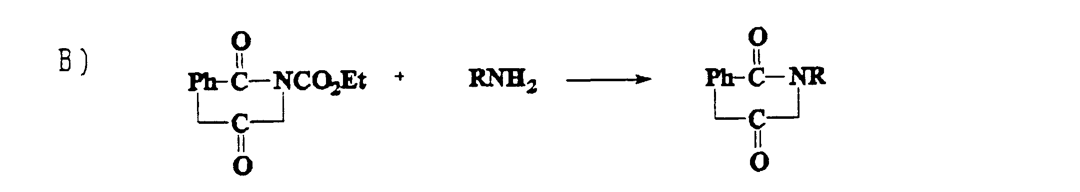

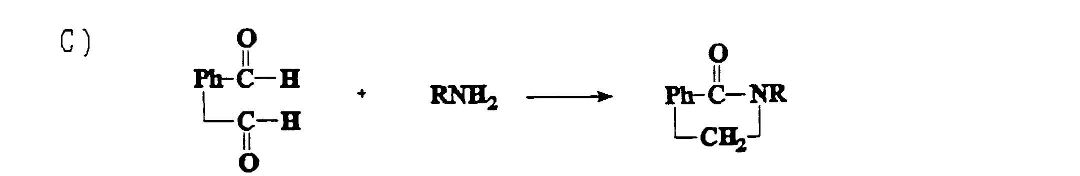

The following examples will serve to further typify the nature of this invention but should not be construed as a limitation in the scope thereof, which scope is defined solely by the appended claims.

### EXAMPLE 1

### 1-Phthalimido-1-(3', 4'-dimethoxyphenyl)propan-1-ol

**a) 3-Amino-3-(3',4'-dimethoxyphenyl)-propan-1-ol.** A solution of 3-amino-3-(3',4'-dimethoxyphenyl)-propionate (4.12 grams, 17.2 mmol) in methanol (50 milliliters) was slowly added to stirred sodium borohydride (6.51 grams, 17.2 mmol). After the initial effervescence had ceased the mixture was refluxed for 1 hour. Reaction progress was monitored by TLC (20% ethyl acetate/hexane, uv) and was complete after 1 hour. The reaction mixture was allowed to cool and then 20 milliliters of water was added. The methanol was removed in *vacuo* resulting in the formation of a gum which was extracted into methylene chloride (3 X 20 milliliters). The combined extracts were dried over magnesium sulfate and concentrated to afford an oil which was refrigerated. A waxy solid formed which was dried *in vacuo* (60 oC, < 1 mm) to afford 3.30 g (86%) of the product as a white solid. ¹H NMR (CDCl₃) δ 6.91-6.78(m, 3H), 4.15-4.04(m, 1H), 3.89(s , 3H), 3.88(s , 3H), 3.84-3.71(s , 2H), 3.91-2.45(broad m, 1H), 1.95-1.78(m , 2H).

**b) 1-Phthalimido-1-(3',4'-dimethoxyphenyl)propan-1-ol.** A mixture of 3-amino-3-(3',4'-dimethoxyphenyl)-propan-1-ol (1.11 grams, 5 mmol) and phthalic anhydride (0.74 grams, 5 mmol) were melted together and stirred for 5 minutes. After cooling, a green/yellow glassy semi solid formed which was stirred in ether to afford 1.62g (95%) of crude product as a white solid. The crude product was purified by flash chromatography (silica gel, 40% ethyl acetate/methylene chloride) to afford 1.25 g (73%) of product as a white solid. ¹H NMR (CDCl₃) δ 7.85-7.63 (m, 4 H), 7.18-7.07 (m, 2 H), 6.86-6.76 (m, 1 H), 5.62-5.49(m, 1 H), 3.88 (s , 3 H), 3.85 (s , 3 H), 3.79-3.63 (m , 2 H), 2.89-2.71 (m , 1 H), 2.64-2.47 (m, 1 H), 1.87-1.73 (br m, 1 H). ¹³C NMR (CDCl₃) δ 168.5, 148.8, 148.6, 133.9, 131.8, 131.7, 123.2, 120.7, 111.6, 110.8, 59.8, 55.0, 55.8, 51.4, 33.9. Anal. Calcd. for C₁₉H₁₉NO₅. Theoretical : C ,66.85 ; H, 5.61; N , 4.10. Found : C,66.70; H , 5.60 ; N , 4.06. HPLC 100%.

### EXAMPLE 2

Tablets, each containing 50 milligrams of active ingredient, can be prepared in the following manner:

| Constituents (for 1000 tablets) | |
|---|---|
| active ingredient | 50.0 g |
| lactose | 50.7 g |
| wheat starch | 7.5 g |
| polyethylene glycol 6000 | 5.0 g |
| talc | 5.0 g |
| magnesium stearate | 1.8 g |
| demineralized water | q.s. |

The solid ingredients are first forced through a sieve of 0.6 mm mesh width. The active ingredient, the lactose, the talc, the magnesium stearate and half of the starch are then mixed. The other half of the starch is suspended in 40 milliliters of water and this suspension is added to a boiling solution of the polyethylene glycol in 100 milliliters of water. The resulting paste is added to the pulverulent substances and the mixture is granulated, if necessary with the addition of water. The granulate is dried overnight at 35°C, forced through a sieve of 1.2 mm mesh width and compressed to form tablets of approximately 6 mm diameter which are concave on both sides.

### EXAMPLE 3

Tablets, each containing 100 milligrams of active ingredient, can be prepared in the following manner:

| Constituents (for 1000 tablets) | |
|---|---|
| active ingredient | 100.0 g |
| lactose | 100.0 g |
| wheat starch | 47.0 g |
| magnesium stearate | 3.0 g |

All the solid ingredients are first forced through a sieve of 0.6 mm mesh width. The active ingredient, the lactose, the magnesium stearate and half of the starch then are mixed. The other half of the starch is suspended in 40 milliliters of water and this suspension is added to 100 milliliters of boiling water. The resulting paste is added to the pulverulent substances and the mixture is granulated, if necessary with the addition of water. The granulate is dried overnight at 35°C, forced through a sieve of 1.2 mm mesh width and compressed to form tablets of approximately 6 mm diameter which are concave on both sides.

### EXAMPLE 4

Tablets for chewing, each containing 75 milligrams of active ingredient, can be prepared in the following manner:

| Composition (for 1000 tablets) | |
|---|---|
| active ingredient | 75.0 g |
| mannitol | 230.0 g |
| lactose | 150.0 g |
| talc | 21.0 g |
| glycine | 12.5 g |
| stearic acid | 10.0 g |
| saccharin | 1.5 g |
| 5% gelatin solution | q.s. |

All the solid ingredients are first forced through a sieve of 0.25 mm mesh width. The mannitol and the lactose are mixed, granulated with the addition of gelatin solution, forced through a sieve of 2 mm mesh width, dried at 50°C and again forced through a sieve of 1.7 mm mesh width. The active ingredient, the glycine and the saccharin are carefully mixed, the mannitol, the lactose granulate, the stearic acid and the talc are added and the whole is mixed thoroughly and compressed to form tablets of approximately 10 mm diameter which are concave on both sides and have a breaking groove on the upper side.

### EXAMPLE 5

Tablets, each containing 10 milligrams of active ingredient, can be prepared in the following manner:

| Composition (for 1000 tablets) | |
|---|---|
| active ingredient | 10.0 g |
| lactose | 328.5 g |
| corn starch | 17.5 g |
| polyethylene glycol 6000 | 5.0 g |
| talc | 25.0 g |
| magnesium stearate | 4.0 g |
| demineralized water | q.s. |

The solid ingredients are first forced through a sieve of 0.6 mm mesh width. Then the active ingredient, lactose, talc, magnesium stearate and half of the starch are intimately mixed. The other half of the starch is suspended in 65 milliliters of water and this suspension is added to a boiling solution of the polyethylene glycol in 260 milliliters of water. The resulting paste is added to the pulverulent substances, and the whole is mixed and granulated, if necessary with the addition of water. The granulate is dried overnight at 35°C, forced through a sieve of 1.2 mm mesh width and compressed to form tablets of approximately 10 mm diameter which are concave on both sides and have a breaking notch on the upper side.

### EXAMPLE 6

Gelatin dry-filled capsules, each containing 100 milligrams of active ingredient, can be prepared in the following manner:

| Composition (for 1000 capsules) | |
|---|---|
| active ingredient | 100.0 g |
| microcrystalline cellulose | 30.0 g |
| sodium lauryl sulphate | 2.0 g |
| magnesium stearate | 8.0 g |

The sodium lauryl sulphate is sieved into the active ingredient through a sieve of 0.2 mm mesh width and the two components are intimately mixed for 10 minutes. The microcrystalline cellulose is then added through a sieve of 0.9 mm mesh width and the whole is again intimately mixed for 10 minutes. Finally, the magnesium stearate is added through a sieve of 0.8 mm width and, after mixing for a further 3 minutes, the mixture is introduced in portions of 140 milligrams each into size 0 (elongated) gelatin dry-fill capsules.

### EXAMPLE 7

A 0.2% injection or infusion solution can be prepared, for example, in the following manner:

| | |
|---|---|
| active ingredient | 5.0 g |
| sodium chloride | 22.5 g |
| phosphate buffer pH 7.4 | 300.0 g |
| demineralized water | to 2500.0 milliliters |

The active ingredient is dissolved in 1000 milliliters of water and filtered through a microfilter. The buffer solution is added and the whole is made up to 2500 milliliters with water. To prepare dosage unit forms, portions of 1.0 or 2.5 milliliters each are introduced into glass ampoules (each containing respectively 2.0 or 5.0 milligrams of active ingredient).

## Claims

1. A compound having the formula: 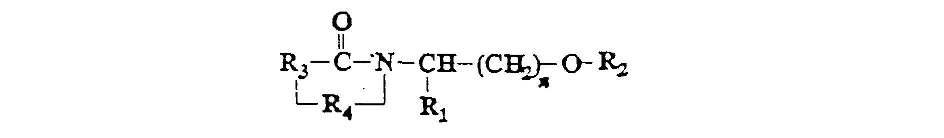 wherein
R¹ is (i) straight, branched, or cyclic, unsubstituted alkyl of 1 to 12 carbon atoms; (ii) straight, branched, or cyclic, substituted alkyl of 1 to 12 carbon atoms; (iii) phenyl; or (iv) phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or halo;
R² is -H or alkyl of 1 to 8 carbon atoms;
R³ is i) ethylene, ii) vinylene, iii) a branched alkylene of 3 to 10 carbon atoms, iv) a branched alkenylene of 3 to 10 carbon atoms, v) cycloalkylene of 4 to 9 carbon atoms unsubstituted or substituted with 1 or more substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, vi) cycloalkenylene of 4 to 9 carbon atoms unsubstituted or substituted with 1 or more substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, or vii) o-phenylene unsubstituted or substituted with 1 or more substituents each selected independently from nitro, cyano, trifluoromethyl, carbethoxy, carbomethoxy, carbopropoxy, acetyl, carbamoyl, acetoxy, carboxy, hydroxy, amino, substituted amino,alkyl of 1 to 4 carbon atoms, alkoxy of 1 to 4 carbon atoms, or halo;
R⁴ is -CX- or -CH₂-;
X is O or S;
and,
*n* is 0,1,2, or 3.

2. The compound of claim 1 wherein R⁴ is -CO- and R¹ is 3,4-diethoxyphenyl.

3. The compound of claim 1 wherein R¹ is 3,4-dimethoxyphenyl.

4. The compound of claim 1 wherein R⁴ is -CO- and R¹ is substituted phenyl.

5. A compound according to any one of claims 1 to 4 for use as a medicament.

6. Use of a compound according to any one of claims 1 to 4 in the manufacture of a medicament for treating conditions associated with high levels of TNFα in a mammal.

7. Use of a compound according to any one of claims 1 to 4 in the manufacture of a medicament as in inhibitor in conditions associated with TNFα-activated retrovirus replication in a mammal.

8. A pharmaceutical composition comprising a compound according to any one of claims 1 to 4.

9. A pharmaceutical composition comprising an amount of a compound according to any one of claims 1 to 4 effective upon single or multiple dosage to inhibit TNFα.

## Revendications

1. Composé de formule: 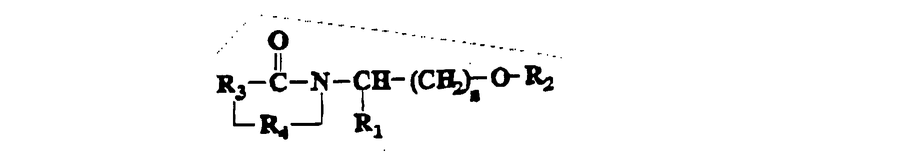 dans laquelle
R¹ est (i) un groupe alkyle linéaire, ramifié ou cyclique, non substitué, ayant de 1 à 12 atomes de carbone; (ii) un groupe alkyle linéaire, ramifié ou cyclique, substitué, ayant de 1 à 12 atomes de carbone; (iii) un groupe phényle; ou (iv) un groupe phényle substitué par un ou plusieurs substituants choisis indépendamment les uns des autres dans le groupe constitué par les radicaux nitro, cyano, trifluorométhyle, carbéthoxy, carbométhoxy, carbopropoxy, acétyle, carbamoyle, acétoxy, carboxy, hydroxy, amino, amino substitué, alkyle de 1 à 10 atomes de carbone, alcoxy de 1 à 10 atomes de carbone, ou halogéno;
R² est -H ou un groupe alkyle de 1 à 8 atomes de carbone;
R³ est i) un éthylène, ii) un vinylène, iii) un alkylène ramifié de 3 à 10 atomes de carbone, iv) un alcénylène ramifié de 3 à 10 atomes de carbone, v) un cycloalkylène de 4 à 9 atomes de carbone, non substitué ou substitué par un ou plusieurs substituants dont chacun est indépendamment choisi parmi un groupe nitro, cyano, trifluorométhyle, carbéthoxy, carbométhoxy, carbopropoxy, acétyle, carbamoyle, acétoxy, carboxy, hydroxy, amino, amino substitué, alkyle de 1 à 4 atomes de carbone, alcoxy de 1 à 4 atomes de carbone, ou halogéno, vi) un cycloalcénylène de 4 à 9 atomes de carbone, non substitué ou substitué par un ou plusieurs substituants dont chacun est choisi indépendamment parmi un groupe nitro, cyano, trifluoroinéthyle, carbéthoxy, carbométhoxy, carbopropoxy, acétyle, carbamoyle, acétoxy, carboxy, hydroxy, amino, amino substitué, alkyle de 1 à 4 atomes de carbone, alcoxy de 1 à 4 atomes de carbone, ou halogéno, ou vii) un *o*-phénylène non substitué ou substitué par un ou plusieurs substituants dont chacun est choisi indépendamment parmi un groupe nitro, cyano, trifluorométhyle, carbéthoxy, carbométhoxy, carbopropoxy, acétyle, carbamoyle, acétoxy, carboxy, hydroxy, amino, amino substitué, alkyle de 1 à 4 atomes de carbone, alcoxy de 1 à 4 atomes de carbone, ou halogéno;
R⁴ est -CX- ou -CH₂-;
X est O ou S;
et,
n vaut 0, 1, 2 ou 3.

2. Composé selon la revendication 1, dans lequel R⁴ est -CO- et R¹ est un groupe 3,4-diéthoxyphényle.

3. Composé selon la revendication 1, dans lequel R¹ est un groupe 3,4-diméthoxyphényle.

4. Composé selon la revendication 1, dans lequel R⁴ est -CO- et R¹ est un groupe phényle substitué.

5. Composé selon l'une quelconque des revendications 1 à 4, destiné à être utilisé comme médicament.

6. Utilisation d'un composé selon l'une quelconque des revendications 1 à 4 dans la fabrication d'un médicament destiné au traitement d'affections associées à des niveaux élevés de TNFα chez un mammifère.

7. Utilisation d'un composé selon l'une quelconque des revendications 1 à 4 dans la fabrication d'un médicament utile en tant qu'inhibiteur dans des affections associées à une réplication rétrovirale activée par le TNFα chez un mammifère.

8. Composition pharmaceutique comprenant un composé selon l'une quelconque des revendications 1 à 4.

9. Composition pharmaceutique comprenant une quantité d'un composé selon l'une quelconque des revendications 1 à 4 qui est efficace en dose unique ou multiple pour inhiber le TNFα.

## Patentansprüche

1. Verbindung der Formel 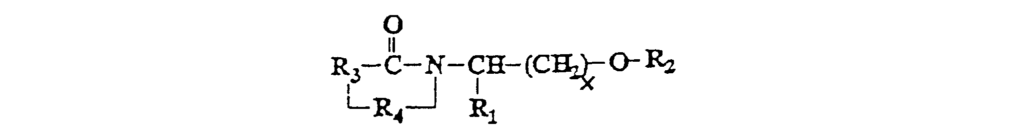 wobei
R¹ (i) ein geradkettiger, verzweigter oder cyclischer unsubstituierter Alkylrest mit 1 bis 12 Kohlenstoffatomen, (ii) ein geradkettiger, verzweigter oder cyclischer substituierter Alkylrest mit 1 bis 12 Kohlenstoffatomen (iii) Phenyl oder (iv) Phenyl, substituiert mit einem oder mehreren Substituenten, der bzw. die jeweils unabhängig vom anderen aus der Gruppe, bestehend aus Nitro, Cyano, Trifluormethyl, Carbethoxy, Carbomethoxy, Carbopropoxy, Acetyl, Carbamoyl, Acetoxy, Carboxy, Hydroxy, Amino, substituiertem Amino, Alkyl mit 1 bis 10 Kohlenstoffatomen, Alkoxy mit 1 bis 10 Kohlenstoffatomen oder Halogen, ausgewählt ist bzw. sind, bedeutet,
R² -H oder einen Alkylrest mit 1 bis 8 Kohlenstoffatomen bedeutet,
R³ i) Ethylen, ii) Vinylen, iii) einen verzweigten Alkylenrest mit 3 bis 10 Kohlenstoffatomen, iv) einen verzweigten Alkenylenrest mit 3 bis 10 Kohlenstoffatomen, v) einen Cycloalkylenrest mit 4 bis 9 Kohlenstoffatomen, unsubstituiert oder substituiert mit 1 oder mehreren Substituenten, der bzw. die jeweils unabhängig voneinander aus Nitro, Cyano, Trifluormethyl, Carbethoxy, Carbomethoxy, Carbopropoxy, Acetyl, Carbamoyl, Acetoxy, Carboxy, Hydroxy, Amino, substituiertem Amino, Alkyl mit 1 bis 4 Kohlenstoffatomen, Alkoxy mit 1 bis 4 Kohlenstoffatomen oder Halogen ausgewählt ist bzw. sind, vi) einen Cycloalkylenrest mit 4 bis 9 Kohlenstoffatomen, unsubstituiert oder substituiert mit 1 oder mehreren Substituenten, der bzw. die jeweils unabhängig voneinander aus Nitro, Cyano, Trifluormethyl, Carbethoxy, Carbomethoxy, Carbopropoxy, Acetyl, Carbamoyl, Acetoxy, Carboxy, Hydroxy, Amino, substituiertem Amino, Alkyl mit 1 bis 4 Kohlenstoffatomen, Alkoxy mit 1 bis 4 Kohlenstoffatomen oder Halogen ausgewählt ist bzw. sind, oder vii) o-Phenylen, unsubstituiert oder substituiert mit 1 oder mehreren Substituenten, der bzw. die jeweils unabhängig voneinander aus Nitro, Cyano, Trifluormethyl, Carbethoxy, Carbomethoxy, Carbopropoxy, Acetyl, Carbamoyl, Acetoxy, Carboxy, Hydroxy, Amino, substituiertem Amino, Alkyl mit 1 bis 4 Kohlenstoffatomen, Alkoxy mit 1 bis 4 Kohlenstoffatomen oder Halogen ausgewählt ist bzw. sind, bedeutet,
R⁴ -CX- oder -CH₂- bedeutet,
X O oder S bedeutet und
*n* 0,1,2 oder 3 bedeutet.

2. Verbindung nach Anspruch 1, wobei R⁴ -CO- bedeutet und R¹ 3,4-Diethoxyphenyl bedeutet.

3. Verbindung nach Anspruch 1, wobei R¹ 3,4-Dimethoxyphenyl bedeutet.

4. Verbindung nach Anspruch 1, wobei R⁴ -CO- bedeutet und R¹ ein substituiertes Phenyl bedeutet.

5. Verbindung nach einem der Ansprüche 1 bis 4 zur Verwendung als Arzneimittel.

6. Verwendung einer Verbindung nach einem der Ansprüche 1 bis 4 zur Herstellung eines Arzneimittels zur Behandlung von Zuständen, die mit hohen TNFα-Gehalten in einem Säuger zusammenhängen.

7. Verwendung einer Verbindung nach einem der Ansprüche 1 bis 4 zur Herstellung eines Arzneimittels als Inhibitor bei Zuständen, die mit TNFα-aktivierter Retrovirusreplikation in einem Säuger zusammenhängen.

8. Pharmazeutische Zusammensetzung, umfassend eine Verbindung nach einem der Ansprüche 1 bis 4.

9. Pharmazeutische Zusammensetzung, umfassend eine Menge einer Verbindung nach einem der Ansprüche 1 bis 4, die nach einzelner oder mehrfacher Dosierung zur Inhibierung von TNFα wirksam ist.
